# EUROPEAN PATENT APPLICATION

(11) **EP 3 488 710 A1**
(43) Date of publication of application: **29.05.2019**
(21) Application number: 18162583.1
(22) Date of filing: 19.03.2018
(51) Int. Cl.: A23L 33/185, A23L 33/00, A23C 11/00, A23C 11/02, A23C 11/06

(54) **NON-DAIRY VEGETABLE-BASED INSTANT YOGURT POWDER**

(30) Priority: 28.11.2017 EP 17204213
(71) Applicant: Cosucra Groupe Warcoing S.A., 7740 Warcoing (BE)
(72) Inventor: Vervacke, Christophe, 7540 Rumillies (BE); Vandaele, Joséphine, 59800 Lille (FR)

(57) **Abstract**

The present invention is situated in the field of food technology. The invention more specifically relates to a non-dairy vegetable-based instant yogurt powder as well as methods for producing this. The invention further provides product derived from such a reconstituted non-dairy vegetable-based yogurt powder such as a non-dairy vegetable-based yogurt as well as methods for producing this.

## Description

### Field of the invention

The present invention is situated in the field of food technology. The invention more specifically relates to a non-dairy vegetable-based instant yogurt powder as well as methods for producing this. The invention further provides product derived from such a reconstituted non-dairy vegetable-based yogurt powder such as a non-dairy vegetable-based yogurt as well as methods for producing this.

### Background of the invention

Yogurt (also yoghurt or yoghourt) is a food produced by bacterial fermentation of milk. The Europe yogurt market is expected to grow. The increasing awareness of health benefits of yogurt acts as a catalyst to the growth of this market. Yogurt consumption is growing rapidly in high-income countries; wherein, low-sugar yogurts are gaining popularity. Pleasure is the main reason that is increasing yogurt consumption, by almost 35%. The demand for yogurt has been increasing from consumers aged above 50 years. This is due to the increasing awareness of the health benefits of yogurt, which is rich in calcium and protein. Some of the health benefits of yogurt include boosting the immune system, combating diarrhoea and constipation, etc. The increasing availability of flavoured yogurt is also a driver of this market. A rise in the cost of production poses a threat to the market. Also, the fat content in yogurt results in health concerns related to obesity.
Yogurt must be maintained at low temperature, between 2 and 8°C and their shelf-life is quite short, approximatively one month. Dairy powder yogurt already exists and presents numerous advantages like long shelf-life, easy to take away, easy to make at home and the satisfaction to make homemade yogurt.
However, milk is one of the most common food allergens. People with an allergy to cow's milk may also be allergic to milk from other animals, including sheep and goats. Within a brief period of time after consuming milk or a milk protein, they may experience the following symptoms: hives, stomach upset, vomiting, bloody stools, especially in infants, and in some extreme cases anaphylaxis, which is a rare, potentially life-threatening reaction that impairs breathing and can send the body into shock.
Some milk alternatives already exist like: almond-, coconut-, rice-, oat- or soy-based products.

In view of all the disadvantages at the health, economic, environmental and nutritional level that are associated with the consumption of dairy yogurt and non-dairy alternatives yogurt already in the market, there is still a need to find a yogurt which is non-dairy and in a form of powder.

### Summary of the invention

It is the merit of the applicant of having discovered that vegetable protein, could surprisingly and advantageously be used in non-dairy vegetable-based instant yogurt powder, while maintaining organoleptic properties, and in particular, obtaining gustatory, olfactory, visual and tactile properties that are at least equivalent, or even better than those made with milk proteins. Particular advantages of non-dairy vegetable-based instant yogurt powder are, that they are dairy-free and are easy to preserve, easy to take away and has a long shelf life compared to non-dairy yogurt and dairy yogurt powder.

According to a first aspect of the present invention, a non-dairy vegetable-based instant yogurt powder comprising vegetable protein is described.

According to a second aspect of the present invention, a non-dairy vegetable-based yogurt comprising a reconstituted non-dairy vegetable-based instant yogurt powder is described.

According to a third aspect of the present invention, the use of a non-dairy vegetable-based instant yogurt powder to produce a non-dairy vegetable-based yogurt is provided.

Hereto, the present invention is in particular captured by any one or any combination of one or more of the below aspects and embodiments and numbered statements 1 to 24.
1. A non-dairy vegetable-based instant yogurt powder comprising vegetable protein in an amount comprised between 2.5 wt.% and 40 wt.% based on the total weight of the non-dairy vegetable based instant yogurt powder.
2. The non-dairy vegetable-based instant yogurt powder according to statement 1, wherein said vegetable protein is protein derived from the family of cereals, oleaginous plants, tuberous plants and pulse, used alone or as a mixture.
3. The non-dairy vegetable-based instant yogurt powder according to statement 2, wherein said vegetable protein is a pulse protein derived from pulses selected from the group comprising: lentils, beans, peas, chickpeas and combinations thereof.
4. The non-dairy vegetable-based instant yogurt powder according to statement 3, wherein said pulse protein is pea protein, more preferably a pea protein isolate.
5. The non-dairy vegetable-based instant yogurt powder according to any one of statements 1 to 4, wherein said vegetable pulse has not been subjected to a hydrolysis process.
6. The non-dairy vegetable-based instant yogurt powder according to any one of statements 1 to 5, wherein said pulse protein possess a degree of hydrolysis of 5 % of lower.
7. The non-dairy vegetable-based instant yogurt powder according to any one of statements 1 to 6, wherein said powder further comprises ferment(s) and/or carbohydrate(s).
8. The non-dairy vegetable-based instant yogurt powder according to statement 7, wherein said ferment(s) are lyophilized.
9. The non-dairy vegetable-based instant yogurt powder according to statement 7 or 8, wherein said ferment(s) are chosen from Lactobacillus, Streptococcus and Bifidobacterium species, alone or as a mixture.
10. The non-dairy vegetable-based instant yogurt powder according to any one of statements 1 to 9, wherein said powder further comprises stabilizer(s), fat(s) and soluble fiber(s).
11. The non-dairy vegetable-based instant yogurt powder according to statement 10, wherein said soluble fiber is selected from: inulin, glucooligosaccharides (GOSs), isomaltooligosaccharides (IMOs), galactooligosaccharides (TOSs), pyrodextrins, polydextrose, branched maltodextrins, indigestible dextrins and soluble oligosaccharides derived from oleaginous plants or protein-producing plants, or combinations thereof.
12. The non-dairy vegetable-based instant yogurt powder according to any one of statements 1 to 11, wherein the non-dairy vegetable based instant yogurt powder is substantially free of dairy-derived ingredients.
13. A non-dairy vegetable-based yogurt comprising the reconstituted non-dairy vegetable-based instant yogurt powder according to any one of statements 1 to 12.
14. The non-dairy vegetable-based yogurt according to statement 13, wherein said yogurt comprises water.
15. The non-dairy vegetable-based yogurt according to statement 13 or 14, wherein the non-dairy vegetable-based yogurt has a pH comprised between 4.2 and 4.8, preferably between 4.3 and 4.7
16. The non-dairy vegetable-based yogurt according to any one of statements 13 to 15, wherein the non-dairy vegetable-based yogurt has a hardness (maximum force) of between 50 and 200g, preferably between 70 and 120g.
17. The non-dairy vegetable-based yogurt according to any one of statements 13 to 16, wherein the non-dairy vegetable-based yogurt has a stability of more than 90%, preferably more that 95%, more preferably of more than 98%.
18. Use of a non-dairy vegetable-based instant yogurt powder according to any one of statement 1 to 12 for producing a non-dairy vegetable-based yogurt.
19. Use of a non-dairy vegetable-based instant yogurt powder according to statement 18 for producing a non-dairy vegetable-based yogurt, wherein said powder is divided in two portions.
20. Use of a non-dairy vegetable-based instant yogurt powder for producing a non-dairy vegetable-based yogurt according to statements 18 or 19, wherein said one of portion comprises vegetable protein, stabilizer(s) and fat(s).
21. Use of a non-dairy vegetable-based instant yogurt powder for producing a non-dairy vegetable-based yogurt according to statements 18 to 20, wherein said one of portion comprises the other ingredients.
22. A process for manufacturing an instant non-dairy vegetable-based instant yogurt powder according to any one of statements 1 to 12, characterized in that it comprises the following steps:
   - mixing powders of pea protein, stabilizer(s), minerals and fat(s) in order to obtain a first powder blend
   - mixing rest of powders ingredients including ferment(s).
23. A process for manufacturing a non-dairy vegetable-based yogurt according to any one of statements 13 to 17, characterized in that it comprises the following steps:
   1. Mixing powders of pea protein, stabilizer(s) and fat(s) to get a first powder blend
   2. Heat the first powder blend with water with a ratio first powder blend/water of 1/8 to boiling and then cool down to get a first mixture
   3. Mixing remaining powders ingredients, including ferments, to get a second powder blend
   4. Mixing the second powder blend with the first mixture to get the final mixture
   5. Place the final mixture in temperature-controlled chamber,
   6. Wait until the final pH is below 5, preferably around 4.5.
   7. When the pH is achieved, the finished product can be stored in a fridge till consumption
24. A process according to statement 23, wherein the temperature of the temperature-controlled chamber is the optimal temperature for the ferments, more preferably between 40 and 45°C.

### Detailed description

Before the present method of the invention is described, it is to be understood that this invention is not limited to particular methods, components, products or combinations described, as such methods, components, products and combinations may, of course, vary. It is also to be understood that the terminology used herein is not intended to be limiting, since the scope of the present invention will be limited only by the appended claims.
As used herein, the singular forms "a", "an", and "the" include both singular and plural referents unless the context clearly dictates otherwise.
The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".
The recitation of numerical ranges by endpoints includes all numbers and fractions subsumed within the respective ranges, as well as the recited endpoints.
The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.
Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.
All references cited in the present specification are hereby incorporated by reference in their entirety. In particular, the teachings of all references herein specifically referred to are incorporated by reference.
Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.
In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.
Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the invention, and form different embodiments, as would be understood by those in the art. For example, in the appended claims, any of the claimed embodiments can be used in any combination.
The following detailed description, therefore, is not to be taken in a limiting sense, and the scope of the present invention is defined by the appended claims.

In the present invention, the term "vegetable protein" denotes all proteins derived from cereals, oleaginous plants, tuberous plants and pulse.
These vegetable proteins can be used alone or as a mixture, chosen from the same family or from different families.

In the present invention, the term "cereals" is intended to mean cultivated plants of the grass family producing edible seeds. Non-limiting examples are wheat, oats, rye, barley, corn, sunflower, sorghum or rice. The cereals are often milled in the form of flour but are also available as grains and sometimes in whole-plant form (fodders).

In the present invention, the term "oleaginous plants" denotes plants cultivated specifically for their seeds or their fruits rich in fats, from which oil for dietary, energy or industrial use is extracted. Non-limiting examples are rapeseed, groundnut, sunflower, soybean, sesame and the castor oil plant.

In the present invention, the term "tuberous plants" is intended to mean all plants comprising storage organs, which are generally underground, which ensure plant survival during the winter season and often plant multiplication by the vegetative process. These organs are bulging owing to the accumulation of storage substances. The organs transformed into tubers may be:
- the root: Non-limiting examples are carrot, parsnip, cassava, konjac,
- the rhizome: Non-limiting examples are potato, Jerusalem artichoke, Japanese artichoke, sweet potato,
- the base of the stalk (more specifically the hypocotyl): Non-limiting examples are kohlrabi, celeriac,
- the root + hypocotyl combination: Non-limiting examples are beetroot, radish.

In the present invention, the term "pulses" is intended to mean the dried seeds of legumes. The four most common pulses are beans, chickpeas, lentils and peas. Lentils, as *Lens Culinaris,* are represented by: Beluga Lentils, Brown Lentils, French Green Lentils, Green Lentils and Red Lentils. Beans, as *Phaseolus Vulgaris,* are represented by: Adzuki Beans, Anasazi Beans, Appaloosa Beans, Baby Lima Beans, Black Calypso Beans, Black Turtle Beans, Dark Red Kidney Beans, Great Northern Beans, Jacob's Cattle Trout Beans, Large Faba Beans, Large Lima Beans, Mung Beans, Pink Beans, Pinto Beans, Romano Beans, Scarlet Runner Beans, Tongue of Fire, White Kidney Beans and White Navy Beans. Peas are represented by: Black-Eyed Peas, Green Peas, Marrowfat Peas, Pigeon Peas, Yellow Peas and Yellow-Eyed Peas. Chickpeas, as *Cicer Arietinum,* are represented by: Chickpea and Kabuli.

In a preferred embodiment, pulse protein is used. In a most preferred embodiment, pea protein is used. Dry peas contain 20-30% lysine rich proteins. Pea proteins are mainly storage protein comprised of albumins and two globulins, legumin and vicilin which proteins may relatively easily solubilized and isolated. In addition, these proteins are characterized by high lysine content, which is deficient in many other proteins of plant origin. Solubility profile of pea protein isolates is similar to other legume proteins and is characterized by high solubility at alkaline pH-s, a minimum solubility at isoelectric point and moderate solubility in acidic medium. They are characterized by a relatively good emulsifying activity.

Pea protein can be prepared in three forms: pea flour, pea protein concentrate, and pea protein isolate. Pea flour is produced by dry milling of dehulled peas. Pea protein concentrate can be produced through the acid leaching procedure traditionally used to produce soy protein concentrates, but it is more economical to use dry separation methods. Pea protein isolate is produced by wet processing methods.
Proteins and starches within non-oilseed legumes such as peas can be efficiently fractionated by dry milling and air classification. Fine grinding results in flours containing populations of particles differentiated by size and density. Air classification of such flours separates the protein (fine fraction) from the starch (coarse fraction). In this dry process, whole or dehulled peas are pin milled to a very fine flour. During milling starch granules remain relatively intact, while the protein matrix is broken down into fine particles. Care must be used in milling to avoid damage to starch granules. The flour is air classified in a spiral air stream into a fine fraction containing approximately 75% of the total protein, but only 25% of the total mass; and a coarse fraction, containing most of the starch granules. The starch and protein fractions are separated in the air classifier based on their differential mass and size. The coarse fraction can be centrifugally separated from the fine fraction and carried into a coarse fraction duct. The fine fraction is carried with the air into the air cyclone. After milling, some protein bodies still adhere to the starch granules and some starch is still imbedded in the protein matrix. The level of adhering protein bodies and agglomerated starch and protein can be reduced by repeated pin milling and air classification. With such a double pass process, a yield of 33-35% total protein fraction (protein content of 56%, dry basis, N x 6.25) was obtained for peas with an original protein content of about 25%.
Highly concentrated protein fractions (protein isolates) and protein concentrates from pea can be prepared by wet processing. The protein separation has been mainly based on solubilization of protein followed by isoelectric precipitation for subsequent recovery. The isoelectric precipitation process for protein isolate production involves milling of the peas, followed by solubilization of the proteins in water, alkali, or acid; then centrifuging to remove the insoluble components. The solubilized proteins are then precipitated at their isoelectric pH. The precipitated protein curd is collected by centrifugation or sieving. The curd is dried as such to yield isoelectric protein isolate or neutralized and dried to yield a cationic-protein isolate.

Protein isolate have a total protein content (expressed in N 6.25) of at least 70% based on the dry matter, at least 75%, at least 80%, at least 85%, at least 90%, for example between 70% and 99%, preferably between 80% and 95%, more preferably between 80% and 90%. In a preferred embodiment, pea protein isolate is used.

In a preferred embodiment, non-hydrolysed vegetable protein is used. The term "non-hydrolysed protein" is equivalent to an "intact" protein, meaning that the protein has not been subjected to a hydrolysis process. However, minor amounts of hydrolysed proteins may be present in the source of non-hydrolysed proteins, or may be added to the formulation, such as additional amino acids, such as, for example leucine, isoleucine, glutamine, arginine, or dipeptides and the like. According to another embodiment, intact protein may only possess a degree of hydrolysis (DH) of 5% of lower, preferably 4%, 3%, 2%, 1% or lower.

In the present invention, the term "non-dairy" encompasses products that are substantially free of all dairy-derived ingredients. Preferably the term covers products wherein no dairy-derived ingredients are actively added during processing or preparation and hence merely trace amounts of dairy-derived ingredients are present within the products. Typically, the non-dairy vegetable-based instant yogurt powder will contain less than about 1 wt.%, such as less than 0.9 wt.%, 0.8 wt.%, 0.7 wt.%, 0.6 wt.%, 0.5 wt.%, 0.4 wt.%, 0.3 wt.%, 0.2 wt.%, 0.1 wt.% or less of dairy-derived ingredients.

In the present invention, the term "ferment" is intended to mean bacteria that will consume sugar in the absence of oxygen. Example of bacteria are Streptococcus, Lactobacillus or Bifidobacterium species. In preferred embodiment, bacteria are chosen from Streptococcus thermophilus, Lactobacillus bulgaricus, Lactobacillus bulgaricus, Lactobacillus acidophilus or Bifidobacterium animalis ssp. Lactis, alone or as a mixture.

In the present invention, the term "carbohydrate" may be for example: starch and maltodextrin from corn, tapioca, wheat or pea, glucose, fructose, saccharose, mono- and disaccharide.

### Other components

According to one of the preferred embodiment of the present invention, the non-dairy vegetable-based yogurt powder comprises at least one stabilizer. The term "stabilizer" denotes compound such as pectin, starch, microcrystalline cellulose (CMC) or hydrocolloid such as modified starch, alone or in combinations thereof.

According to one of the preferred embodiments of the present invention, the non-dairy vegetable-based yogurt powder comprises at least one fat in a powder form. The fat in a powder form component, may be for example include: animal fats (butter, lard, tallow, chicken fat, and fish oils), cocoa butter and cocoa butter alternatives, lauric oils (coconut, palm kernel), olive oil, palm oil, rapeseed (canola) oil, soybean oil, sunflower seed oil, other vegetable oils or single cell oils; hydrogenated or not; and combinations thereof.

According to one preferred embodiment of the present invention, the non-dairy vegetable-based yogurt powder comprises at least one soluble vegetable fiber.

Preferably, said soluble fiber of vegetable origin is chosen from the group consisting of fructans, including inulin, glucooligosaccharides (GOSs), isomaltooligosaccharides (IMOs), galactooligosaccharides (TOSs), pyrodextrins, polydextrose, branched maltodextrins, indigestible dextrins and soluble oligosaccharides derived from oleaginous plants or protein-producing plants.

As used herein, the term "inulin" refers to a mixture of oligo- and/or polysaccharides of fructose which may have a terminal glucose. Inulins belong to a class of fibers known as fructans. In an embodiment, inulin can be represented, depending from the terminal carbohydrate unit, by the general formulae GFn and/or Fm, wherein G represents a glucose unit, F represents a fructose unit, n is an integer representing the number of fructose units linked to the terminal glucose unit, and m is an integer representing the number of fructose units linked to each other in the carbohydrate chain, preferably wherein n is at least 2, and m is at least 2. Inulins for use in the present invention encompass inulins with a terminal glucose which are also referred as alpha-Dglucopyranosyl-[beta-D-fructofuranosyl](n-1)-D-fructofuranosides, as well as inulins without glucose which are also referred as beta-D-fructopyranosyl-[D-fructofuranosyl](n1)-D-fructofuranosides. Inulins for use in the present invention can also encompass branched inulin. Inulins for use in the present invention can also encompass the hydrolysis products of inulins such as fructo-oligosaccharides (FOS), also called oligofructoses, which are fructose oligomers with a DP of ≤ 20, and they can also encompass fructo-oligosaccharides ending with a terminal glucose with a DP of 3-5 synthesized from sucrose. Preferably said fructo-oligosaccharides have an average DP by number of at least 3 and at most 7. Suitable saccharide chains of inulin from plant origin for use in the invention can have a DP ranging from 2 to about 100. Inulin can be a liquid or a powder product.

The soluble fiber in the non-dairy vegetable-based yogurt powder may be used as a source of fiber, for nutritional value or in order to improve the quality of the non-dairy vegetable-based yogurt in term of firmness, stability or creamy texture.

Additives such as flavours, colorants, thickeners, gelling agents, emulsifiers, bulking agents, minerals and sweeteners may also be found in the non-dairy vegetable-based yogurt powder.

In a first aspect of the present invention, the non-dairy vegetable-based yogurt powder comprises vegetable protein in an amount comprised between 2.5 wt.% and 40 wt.% based on the total weight of the non-dairy vegetable based instant yogurt powder, preferably the non-dairy vegetable-based yogurt powder comprises vegetable protein in an amount of at least 2.5 wt.%, at least 3.0 wt.%, at least 3.5 wt.%, at least 4.0 wt.%, at least 4.5 wt.%, at least 5.0 wt.%, at least 5.5 wt.%, at least 6.0 wt.%, at least 6.5 wt.%, at least 7.0 wt.%, at least 7.5 wt.%, at least 8.0 wt.%, at least 8.5 wt.%, at least 9.0 wt.%, at least 9.5 wt.%, at least 10.0 wt.%, at least 10.5 wt.%, at least 11.0 wt.%, at least 11.5 wt.%, at least 12.0 wt.%, at least 12.5 wt.%, at least 13.0 wt.%, at least 13.5 wt.%, at least 14.0 wt.%, at least 14.5 wt.%, at least 15.0 wt.%, at least 15.5 wt.%, at least 16.0 wt.%, at least 16.5 wt.%, at least 17.0 wt.%, at least 17.5 wt.%, at least 18.0 wt.%, at least 18.5 wt.%, at least 19.0 wt.%, at least 19.5 wt.%, at least 20.0 wt.%, at least 20.5 wt.%, at least 21.0 wt.%, at least 21.5 wt.%, at least 22.0 wt.%, at least 22.5 wt.%, at least 23.0 wt.%, at least 23.5 wt.%, at least 24.0 wt.%, at least 24.5 wt.%, at least 25.0 wt.%, at least 25.5 wt.%, at least 26.0 wt.%, and at most 40 wt.%, at most 39.5 wt.%, at most 39.0 wt.%, at most 38.5 wt.%, at most 38.0 wt.%, at most 37.5 wt.%, at most 37.0 wt.%, at most 36.5 wt.%, at most 36.0 wt.%, at most 35.5 wt.%, at most 35.0 wt.%, at most 34.5 wt.%, at most 34.0 wt.%, at most 33.5 wt.%, at most 33.0 wt.%, at most 32.5 wt.%, at most 32.0 wt.%, at most 31.5 wt.%, at most 31.0 wt.%, at most 30.5 wt.%, at most 30.0 wt.%, at most 29.5 wt.%, at most 29.0 wt.%, at most 28.5 wt.%, at most 28.0 wt.%, at most 27.5 wt.%, at most 27.0 wt.%, at most 26.5 wt.%, preferably between 10.0 wt.% and 35.0 wt.% and more preferably between 15.0 wt.% and 30.0 wt.%.

In an embodiment, the pH of the reconstituted non-dairy vegetable-based yogurt is comprised between 4.2 and 4.8, more preferably around 4.3 and 4.7.

In an embodiment, the reconstituted non-dairy vegetable-based yogurt has a hardness (expressed as maximum force) of between 50 and 200g, preferably between 70 and 120g.

In an embodiment, the reconstituted non-dairy vegetable-based yogurt has a stability of more than 90%, preferably more that 95%, more preferably of more than 98%.

### Protocols

### pH measurement

The pH of the reconstituted non-dairy vegetable yogurt is measured in a beaker of 30 ml, at room temperature, with a Mettler Toledo InLab Solids Pro-ISM probe using a Knick Portavo apparatus.

### Hardness

The hardness is measured with a Texture analyser Lloyd model TA1.
The hardness of the yogurt was measured with these parameters:
- Load on cell: 50 N
- Compression test with a probe Cone P45C
- Speed: 1 mm/sec (20mm),
The hardness is the maximum force in g (after 15 mm of compression).
The measurement was done on a glass pot of 150ml; a diameter of 65mm and a height of 5,5cm.

### Stability

The stability was measured following this protocol:
- Fill a centrifuge tube
- Centrifuge 10 min speed 3 (1800G)
- See if mixture is stable or not, measure the % of the different phases with a ruler.

### Rheolaser analysis

The apparatus used is a Rheolaser master, Micro rheology, purchased by Formulaction. The technology embedded in Rheolaser Master is based on a dynamic light scattering technique, cell diffusing wave spectroscopy. The protocol measurement is presented in the user guide: RheolaserMaster_User_Guide_1_6.
With this apparatus, parameters like Elasticity index, Solid Liquid Balance (SLB, ratio liquid/solid) and Macroscopic Viscosity Index may be recorded.

### Examples

### Example 1: Non-dairy pea-based yogurt

The ingredients are listed in table 1. A blend is made with pea protein isolate (source: Pisane®, sold by Cosucra Group Warcoing), pectin, calcium and fat powder. The water is weighed in a saucepan and the blend is dispersed in water with a whip. The mixture is heated to boiling and then cooled down to room temperature (about 25°C). The remaining ingredients are blended and then mixed together with the first mixture with a whip. The solution is then put into pots and the pots are placed in a temperature-controlled chamber (Temperature between 40°C and 45°C). The fermentation will last 12 hours. The yogurt is placed in the refrigerator until it is consumed.

**Table 1**

| **Ingredients/trials** | **Quantity** |
|---|---|
| Water | 77.85 |
| Pea protein isolate | 4.60 |
| Crystal sugar | 9.00 |
| Inulin | 2.20 |
| Vegetable fat powder¹ | 4.40 |
| Pectin² | 0.30 |
| Dicalcium phosphate anhydrous³ | 0.38 |
| Ferments⁴ | 1.20 |
| Flavour exotic fruits⁵ | 0.07 |
| **Total** | **100** |

| | |
|---|---|
| ¹ Vana-Crema 78T ² Genu Texturizer type YA-100 ³ Prayphos DCPA 308 FG ⁴ Lyophilised ferments Natali (Streptococcus Thermophilus) ⁵ Exotic Fruits Flavor 502448 TP0551, Firmenich | |

A thermal treatment is needed for good solubilization of pea proteins, but ferments must not be heated above 60°C. Therefore, ingredients should be included into two packets. As the product is intended to be reconstituted at home, a "home-made" process has been developed, with everyday equipment. An electric yogurt maker was used.

### Results:

1) Visual aspect: stable, no cracking, colour beige
2) Stability: 100%
3) pH: 4,46
4) Texture analysis with Lloyd:
   Force max/Hardness: 83.91g

## Claims

1. A non-dairy vegetable-based instant yogurt powder comprising vegetable protein in an amount comprised between 2.5 wt.% and 40 wt.% based on the total weight of the non-dairy vegetable based instant yogurt powder.

2. The non-dairy vegetable-based instant yogurt powder according to claim 1, wherein said vegetable protein is protein derived from the family of cereals, oleaginous plants, tuberous plants and pulse, used alone or as a mixture.

3. The non-dairy vegetable-based instant yogurt powder according to claim 2, wherein said vegetable protein is a pulse protein derived from pulses selected from the group comprising: lentils, beans, peas, chickpeas and combinations thereof.

4. The non-dairy vegetable-based instant yogurt powder according to claim 3, wherein said pulse protein is pea protein, more preferably a pea protein isolate.

5. The non-dairy vegetable-based instant yogurt powder according to any one of claims 1 to 4, wherein said powder further comprises ferment(s) and/or carbohydrate(s).

6. The non-dairy vegetable-based instant yogurt powder according to claim 5, wherein said ferment(s) are chosen from Lactobacillus, Streptococcus and Bifidobacterium species, alone or as a mixture.

7. The non-dairy vegetable-based instant yogurt powder according to any one of claims 1 to 6, wherein said powder further comprises stabilizers(s), fat(s) and soluble fiber(s).

8. The non-dairy vegetable-based instant yogurt powder according to claim 7, wherein said soluble fiber is selected from: inulin, glucooligosaccharides (GOSs), isomaltooligosaccharides (IMOs), galactooligosaccharides (TOSs), pyrodextrins, polydextrose, branched maltodextrins, indigestible dextrins and soluble oligosaccharides derived from oleaginous plants or protein-producing plants, or combinations thereof.

9. A non-dairy vegetable-based yogurt comprising the reconstituted non-dairy vegetable-based instant yogurt powder according to any one of claims 1 to 8.

10. The non-dairy vegetable-based yogurt according to claim 9, wherein said yogurt comprises water.

11. The non-dairy vegetable-based yogurt according to claims 9 or 10, wherein the non-dairy vegetable-based yogurt has a pH comprised between 4.2 and 4.8, preferably between 4.3 and 4.7.

12. The non-dairy vegetable-based yogurt according to any one of claims 9 to 11, wherein the non-dairy vegetable-based yogurt has a hardness (maximum force) of between 50 and 200g, preferably between 70 and 120g.

13. The non-dairy vegetable-based yogurt according to any one of claims 9 to 12, wherein the non-dairy vegetable-based yogurt has a stability of more than 90%, preferably more that 95%, more preferably of more than 98%.

14. Use of a non-dairy vegetable-based instant yogurt powder according to any one of claims 1 to 8 for producing a non-dairy vegetable-based yogurt.

15. A process for manufacturing an instant non-dairy vegetable-based yogurt powder according to any one of claims 1 to 13, **characterized in that** it comprises the following steps:
- mixing powders of pea protein, stabilizer(s), minerals and fat(s) in order to obtain a first powder blend
- mixing rest of powders ingredients including ferment(s).

16. A process for manufacturing a non-dairy vegetable-based yogurt according to any one of claims 9 to 13, **characterized in that** it comprises the following steps:
1. Mixing powders of pea protein, stabilizer(s) and fat(s) to get a first powder blend
2. Heat the first powder blend with water with a ratio first powder blend/water of 1/8 to boiling temperature and then cool down to get a first mixture
3. Mixing remaining powder ingredients, including ferments, to get a second powder blend
4. Mixing the second powder blend with the first mixture to get the final mixture
5. Place the final mixture in temperature-controlled chamber,
6. Wait until the final pH is below 5, preferably around 4.5.
7. When the pH is achieved, the finished product can be stored in a fridge till consumption
